# EUROPÄISCHE PATENTSCHRIFT

(11) **EP 2 219 712 B1**
(45) Veröffentlichungstag und Bekanntmachung des Hinweises auf die Patenterteilung: **06.11.2013**
(21) Anmeldenummer: 08850398.2
(22) Anmeldetag: 12.11.2008
(51) Int. Cl.: A61M 5/315, A61M 5/20, A61M 5/24

(54) **DREHBARE FÜHRUNGSHÜLSE MIT ÜBERSPANNUNGSGESICHERTER FEDER**
ROTATABLE GUIDING SLEEVE COMPRISING AN OVERLOAD-PROTECTED SPRING
DOUILLE DE GUIDAGE PIVOTANTE À RESSORT PROTÉGÉ CONTRE TOUTE SURTENSION

(30) Priorität: 12.11.2007 DE 102007053743
(43) Veröffentlichungstag der Anmeldung: 25.08.2010
(73) Patentinhaber: Tecpharma Licensing AG, 3401 Burgdorf (CH)
(72) Erfinder: HIRSCHEL, Jürg, CH-5000 Aarau (CH); MOSER, Ulrich, CH-3412 Heimiswil (CH); SCHRUL, Christian, CH-3421 Lyssach (CH); TSCHIRREN, Markus, CH-3422 Kirchberg (CH)
(86) Internationale Anmeldenummer: PCT/EP2008/009543
(87) Internationale Veröffentlichungsnummer: WO 2009/062685

(56) Entgegenhaltungen:
- WO-A-02/053214
- US-A1- 2006 276 754

## Beschreibung

Die vorliegende Erfindung bezieht sich auf eine Injektionsvorrichtung, welche es ermöglicht, eine in der Injektionsvorrichtung zum Beispiel in einer Ampulle enthaltene Substanz nach Einstellung einer Dosis an der Injektionsvorrichtung dosiert abzugeben, wobei die eingestellte Dosis einfach korrigiert werden können soll. Insbesondere bezieht sich die Erfindung auf eine Dosiseinstellungsvorrichtung zur Verwendung in Kombination mit einem fluidgefüllten Behälter, welche vorzugsweise auf eine wiederholte Abgabe von einzeln eingestellten Fluiddosen aus dem Behälter ausgelegt ist.

Eine Dosiseinstellvorrichtung, welche in zwei Richtungen beweglich ist, ist aus der EP 1 351 732 B1 und der DE 2 951 321 4 U1 bekannt.

Es ist eine Aufgabe der vorliegenden Erfindung eine Dosiseinstellvorrichtung für eine Injektionsvorrichtung vorzuschlagen, welche eine Dosiskorrektur, also z. B. ein Zurücksetzen einer zu groß eingestellten Dosis, auf einfache Art ermöglicht.

Diese Aufgabe wird durch die Dosiseinstellvorrichtung oder Dosiervorrichtung gemäß Anspruch 1 gelöst. Vorteilhafte Ausführungsformen sind in den abhängigen Ansprüchen definiert.

Eine erfindungsgemäße Dosiervorrichtung für eine Injektionsvorrichtung und bevorzugt für eine Auto-Injektionsvorrichtung, bei welcher die Ausschüttung einer Dosis nach Auslösung des Ausschüttvorganges automatisch erfolgt, weist ein Dosierelement, wie z. B. eine Dosierhülse auf, welches vorzugsweise relativ zur Dosiervorrichtung oder einer mit der Dosiervorrichtung verbundenen Injektionsvorrichtung zur Einstellung einer Dosis drehbar ist. Dabei kann das Dosierelement relativ zur Dosiervorrichtung oder Injektionsvorrichtung drehbar gelagert sein, wobei das Dosierelement bevorzugt axial unverschiebbar gelagert ist. Es ist jedoch auch möglich das Dosierelement zum Beispiel durch eine Gewindeführung in axialer Richtung beweglich zu lagern. Das Dosierelement dient zur Einstellung einer Dosis, wobei vorzugsweise ein linearer Zusammenhang zwischen Einstellweg oder Einstellwinkel des Dosierelements und der eingestellten Dosis des aus der Injektionsvorrichtung abzugebenden Fluids besteht. Weiterhin ist ein Führungselement vorgesehen, welches zum Beispiel beim Einstellen einer Dosis durch eine Drehung des Dosierelementes drehfest mit der Dosiervorrichtung oder der Injektionsvorrichtung verbunden ist. Das Führungselement, wie zum Beispiel eine Führungshülse, dient zum Führen eines Kolbens oder einer Gewindestange, welche zum Beispiel durch ein Innengewinde des Führungselementes geführt wird und welche sich zum Abgeben einer Dosis, geführt durch das vorzugsweise in axialer Richtung der Injektionsvorrichtung nicht bewegbare Führungselement, in distale Richtung der Injektionsvorrichtung bewegt oder schraubt, um zum Beispiel auf einen Stopfen zu drücken und so eine Abgabe eines Fluids zu bewirken. Zum Einstellen einer Dosis wird das Dosierelement relativ zum Führungselement bewegt oder gedreht, also zum Beispiel verschoben, werden. Erfindungsgemäß ist das Führungselement so ausgelegt und in der Dosiervorrichtung oder Injektionsvorrichtung vorgesehen und mit dem Dosierelement gekoppelt, dass bei einer Einstell- oder Drehbewegung zur Korrektur der mit dem Dosierelement eingestellten Dosis, also beispielsweise einer Drehbewegung in Gegenrichtung der Aufdosier-Drehrichtung, das Führungselement mit dem Dosierelement mitbewegt wird. Beispielsweise kann das Führungselement so in der Dosiervorrichtung oder Injektionsvorrichtung angeordnet oder gelagert sein, dass eine Korrektur-Bewegung oder Gegen-Drehbewegung des Dosierelementes auf das Führungselement übertragen wird, sodass vorzugsweise das Dosierelement und das Führungselement während oder für die Durchführung eines Rückdreh- oder Korrektur-Einstellvorganges gekoppelt und zum Beispiel relativ zueinander verdrehgesichert und / oder verschiebegesichert sind.

Die erfindungsgemäße Möglichkeit das Führungselement zur Dosiskorrektur freizugeben und nicht fest mit der Injektionsvorrichtung oder Dosiervorrichtung zu verbinden ermöglicht eine Dosiskorrektur, bei welcher im Wesentlichen kein Eingriff im Bereich der Dosiervorrichtung vorgenommen werden muss. Folglich ist es erfindungsgemäß zum Beispiel nicht erforderlich, dass die Kopplung zwischen dem Dosierelement und der Gewindestange aufgehoben wird, wodurch der konstruktive Aufbau der Dosiervorrichtung und somit der Injektionsvorrichtung vereinfacht wird.

Vorzugsweise weist die Dosiervorrichtung eine Gewindestange und bevorzugt eine Zahn-Gewindestange auf, bei welcher in Umfangsrichtung ein durchgehendes oder aus Teilstücken bestehendes Gewinde vorgesehen ist, wobei das Gewinde oder einzelne Gewindeabschnitte eine Verzahnung aufweisen, die zum Beispiel aus in axialer Richtung verlaufenden Zähnen oder Zahn-Teilstücken besteht. Diese Gewindestange wird in einem Innengewinde des Führungselementes geführt, wobei das Führungselement vorzugsweise mindestens ein Rastelement aufweist, welches mit einer Zahnung der Zahn-Gewindestange so zusammenwirken kann, dass eine relative Drehbewegung der Zahn-Gewindestange zum Führungselement in eine Richtung (z. B. bei der Substanzabgabe) möglich ist und in Gegenrichtung (z. B. bei der Dosiskorrektur) gesperrt oder blockiert wird.

Vorteilhaft ist das Dosierelement mit einem Koppelelement verdrehsicher gekoppelt, sodass eine Drehung des Dosierelementes auf das Koppelelement übertragen werden kann. Dabei kann das Koppelelement auch zum Beispiel auf seiner Außenseite in Umfangsrichtung eine Beschriftung aufweisen, an welcher zum Beispiel die eingestellte Dosis im Bereich eines Sichtfensters abgelesen werden kann, sodass das Koppelelement zum Beispiel als Anzeigetrommel wirkt. Das Koppelelement kann in axialer Richtung relativ zum Dosierelement beweglich sein und beispielsweise ein Innengewinde oder Außengewinde aufweisen, welches in einem entsprechendem Gegengewinde der Injektionsvorrichtung oder Dosiervorrichtung geführt ist, um eine axiale Verschiebung des Koppelelementes bei einer Drehung des Koppelelementes zu bewirken.

Das Koppelelement und / oder das Einstellelement können ein Rastelement, wie zum Beispiel einen oder mehrere beispielsweise radial nach innen vorgespannte Schnapparme aufweisen, welche in eine Zahnung der Zahn-Gewindestange eingreifen können, um eine Drehbewegung der Zahn-Gewindestange relativ zum Dosierelement oder Koppelelement in eine Richtung zu ermöglichen und in Gegenrichtung zu verhindern.

Vorzugsweise ist verdrehgesichert mit dem Koppelelement und / oder verdrehgesichert mit dem Einstellelement ein Federspannelement vorgesehen, welches durch eine Drehung des Dosierelementes gedreht wird. Dabei ist vorteilhaft ein Federelement, wie beispielsweise eine Spiralfeder oder aufgewickelte Blattfeder, zwischen dem Federspannelement und dem Führungselement vorgesehen und vorzugsweise mit mindestens einem dieser Elemente fest und mit dem anderen lösbar verbunden, so dass das Federelement durch eine relative Drehbewegung zwischen dem Federspannelement und dem Führungselement gespannt werden kann. Es ist auch möglich, dass das Dosierelement direkt mit dem Federspannelement gekoppelt oder verbunden oder damit einteilig ausgebildet ist.

Das Führungselement ist vorzugsweise so in der Dosiervorrichtung oder Injektionsvorrichtung gelagert, dass es bezüglich eines in eine Richtung wirkenden Drehmoments verdrehgesichert ist und in die entgegengesetzte Drehrichtung bewegt werden kann. Hierzu können beispielsweise ein oder mehrere radial nach außen gerichtete gespannte Schnapparme vorgesehen sein, welche beispielsweise in eine Innenverzahnung der Dosiervorrichtung oder Injektionsvorrichtung eingreifen und so eine nur einseitig wirkende Verdrehsicherung realisieren. Falls das Einstellelement z. B. in axiale Richtung verschiebbar ist, ist es vorteilhaft, diese einseitige Sicherung bezüglich dieser Verschiebung vorzusehen.

Vorteilhaft ist ein Auslöseknopf vorgesehen, welcher beispielsweise gegen die Federkraft einer in Einschubrichtung des Auslöseknopfes wirkenden Kraft eingedrückt werden kann, um die Kopplung zwischen dem Federelement und der Zahn-Gewindestange aufzuheben, sodass die Zahn-Gewindestange zum Beispiel nicht mehr durch Schnapparme des Führungselementes gehalten wird, aber noch in dem Gewinde des Führungselementes geführt wird, so dass sich die beim Einstellvorgang durch Drehen der Dosierhülse aufgezogene oder gespannte Feder entspannen kann und eine Drehbewegung des Federspannelementes und optional damit verdrehgesichert gekoppelter Elemente relativ zum Führungselement bewirkt, wobei die Federspannhülse und / oder optional verdrehgesichert damit gekoppelte Elemente zum Beispiel mittels einer oder mehrerer Schnapparme diese Drehbewegung auf die Zahn-Gewindestange übertragen, welche sich somit, geführt durch das Gewinde des Führungselementes in der Dosiervorrichtung, in axiale Richtung und bevorzugt in distale Richtung der Injektionsvorrichtung bewegt.

Des Weiteren bezieht sich der Erfindung auf eine Injektionsvorrichtung mit einer wie oben beschriebenen Dosiervorrichtung.

Vorzugsweise ist die aus dem Dosierelement und Führungslement und optional auch Koppelelement und / oder Federspannelement gebildete Dosiervorrichtung vollständig oder mit einzelnen Komponenten relativ zur Injektionsvorrichtung drehbar. Dabei sind vorzugsweise ein oder mindestens zwei einseitig gerichtete Verdrehsicherungen vorgesehen, welche eine Verdrehung des die Verdrehsicherung tragenden Elements in eine Richtung ermöglichen und in Gegenrichtung sperren.

Nach einem weiteren Aspekt bezieht sich die Erfindung auf ein Verfahren zum Korrigieren einer mit einer Dosiervorrichtung eingestellten Dosis, wobei die Dosis durch Bewegung oder Drehung eines Dosierelementes relativ zu einem Führungselement eingestellt wird und wobei das Führungselement beim Korrigieren der Dosis durch Bewegen oder Drehen des Dosierelementes in Gegenrichtung mit dem Dosierelement bewegt oder mitgedreht wird.

Es ist eine weitere Aufgabe der Erfindung ein Federelement für eine Injektionsvorrichtung vorzuschlagen, welches nicht überspannt oder durch Überspannung beschädigt oder zerstört werden kann.

Diese Aufgabe wird durch eine Überlastsicherung für ein Federelement gemäß dem unabhängigen Anspruch gelöst. Vorteilhafte Ausführungsformen ergeben sich aus den abhängigen Ansprüchen.

Eine Überlastsicherung für ein Federelement einer Dosiervorrichtung oder Injektionsvorrichtung weist ein Federhaltelement auf, wie zum Beispiel ein Führungselement oder eine Führungshülse, welches die Feder relativ zur Dosiervorrichtung oder Injektionsvorrichtung hält. Des weiteren ist ein Federspannelement, wie zum Beispiel eine Federspannhülse oder ein als Federspannelement dienendes Dosierelement vorgesehen, welches zum Spannen der Feder dient, wobei das Federelement vorzugsweise zwischen dem Federhaltelement und dem Federspannelement angeordnet ist. Dabei kann das Federelement an mindestens einem dieser Elemente, also zum Beispiel am Federhalteelement oder am Federspannelement, unlösbar befestigt sein. Erfindungsgemäß sind in dem Federspannelement und / oder dem Federhaltelement ein oder mehr Federhaltebereiche vorgesehen, an oder in welchen ein Halteelement der Feder lösbar gehalten werden kann. Das Federhaltelement der Feder kann sich aus einem der Federhaltebereiche lösen und zum Beispiel zu einem benachbarten Federhaltebereich bewegen, in welchem die Feder gehalten wird, wenn das Federelement zum Beispiel aufgrund des Aufziehens oder Spannens des Federelementes verformt wurde und somit eine Beschädigung droht.

Es ist auch möglich nur einen Federhaltebereich vorzusehen, so dass im Fall eines z. B. zylinderförmigen Federspannelements die Feder bei drohender Überspannung um eine volle Drehung entspannt wird.

Somit ist es möglich ein Überspannen und folglich ein Beschädigen der Feder, welche sich bei zu starkem Spannen selbsttätig entspannen kann, zu verhindern.

Das Halteelement der Feder kann zum Beispiel ein vorstehender Abschnitt, vorzugsweise an einem inneren oder äußerem Endbereich einer aufgerollten Spiralfeder sein, welcher in zum Beispiel rillenförmig ausgebildete Haltebereiche eingreifen kann.

Wird das Federelement zum Beispiel an einem Innenbereich der aufgerollten Feder mit dem Federhalteelement verbunden, so ist vorzugsweise der mindestens eine Federhaltebereich des Federspannelementes außen angeordnet, also in dem Bereich, in dem welchem eine Spiralfeder eine Wicklung mit größerem oder größtem Radius aufweist. Bei umgekehrter Anordnung, wenn die Feder außen gehalten wird, kann der Federhaltebereich auch innen und zum Beispiel auch innerhalb der Feder selbst angeordnet sein und beispielsweise eine zylinderförmiges Teilstück sein, welches an seiner Umfangsrichtung beispielsweise in axiale Richtung verlaufende Rillen oder Nuten aufweist, die als Federhaltebereiche dienen.

Die Erfindung bezieht sich des Weiteren auf eine Überlastsicherung für ein Federelement in einer wie oben beschriebenen Dosiervorrichtung und bevorzugt in einer wie oben beschriebenen Injektionsvorrichtung.

Gemäß einem weiteren Aspekt bezieht sich die Erfindung auf ein Verfahren zum Verhindern einer Überspannung eines Federelementes und insbesondere einer Spiralfeder, wobei die Spiralfeder mit einem Federhaltelement fest verbunden ist und mit einem Federspannelement lösbar verbunden ist, wobei die lösbare Verbindung, zum Beispiel durch die Verformung der Feder bei zu starkem Spannen, selbsttätig gelöst wird, um für ein zumindest teilweises Entspannen der Feder zu sorgen, so dass eine Überspannung und folglich eine Beschädigung der Feder verhindert wird.

Die Erfindung wird anhand von Ausführungsbeispielen näher beschrieben. Es zeigen:
- Figur 1A: einen Querschnitt durch eine Dosiervorrichtung einer Injektionsvorrichtung;
- Figur 1B: einen Querschnitt der in Figur 1A gezeigten Dosiervorrichtung entlang der Linie A-A;
- Figur 2: eine perspektivische Querschnittsansicht einer Injektionsvorrichtung mit der in Figur 1 gezeigten Dosiervorrichtung;
- Figur 3A: einen Querschnitt entlang der Linie B-B in Figur 1B zur Erläuterung einer Überlastsicherung; und
- Figur 3B: eine perspektivische Ansicht des überlastgesicherten Spannmechanismus.

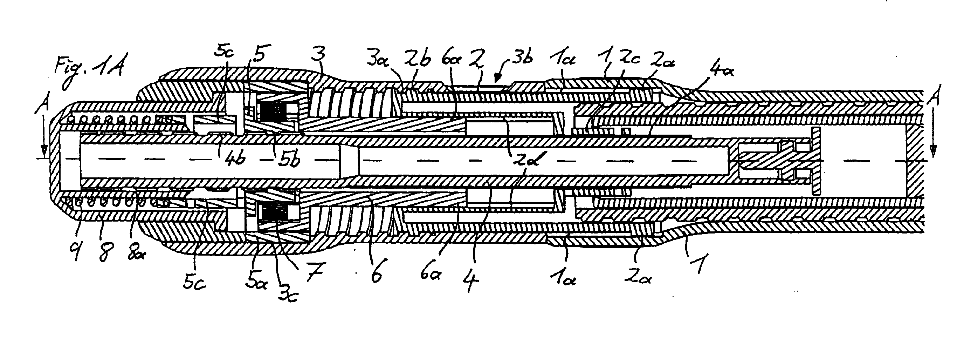

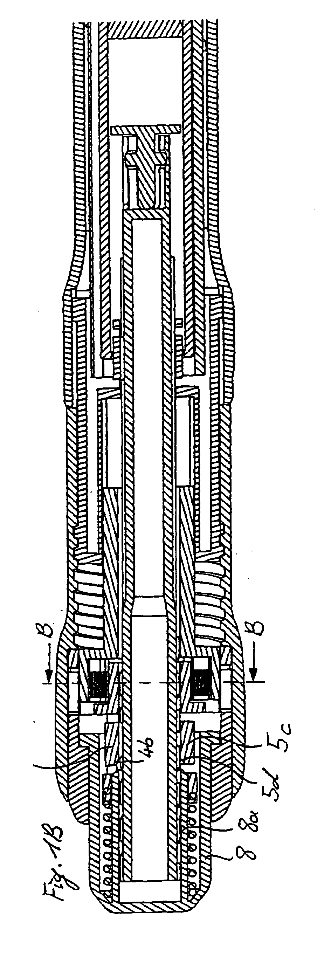

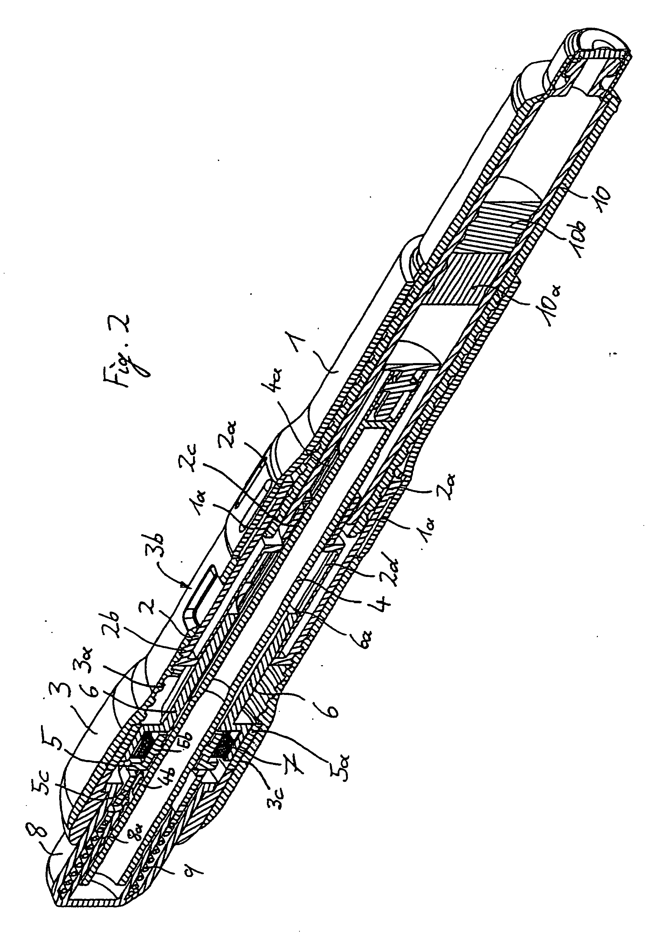

Die Figuren 1A und 1B zeigen eine Dosiereinheit eines in Figur 2 gezeigten Pens bzw. einer Injektionsvorrichtung, welche als so genannter Auto-Pen ausgebildet ist. Die Ausschüttung erfolgt nach Drücken des Auslöseknopfes 8 automatisch mittels einer Feder 7, welche während des Einstellvorganges zum Beispiel durch Drehen der Dosierhülse 1 vorgespannt wird.

Eine Dosierhülse 1 ist axial unverschiebbar aber frei drehbar auf einem Gehäuse 3 der Injektionsvorrichtung vorgesehen.

Die Dosierhülse 1 hat auf der Innenseite in axialer Richtung verlaufend vier Nuten 1a, in welche radial nach außen von der Anzeigetrommel 2 vorstehende Stege 2a ragen. Die Anzeigetrommel 2 ist somit mit der Dosierhülse 1 verdrehgesichert gekoppelt, kann aber in axialer Richtung relativ zur Dosierhülse 1 verschoben werden.

An der proximalen oder hinteren Seite der Anzeigetrommel 2 ist ein Außengewinde 2b vorgesehen, welches in ein Innengewinde 3a des Gehäuses 3 eingreift. Wird die Dosierhülse 1 zum Einstellen einer Dosis gedreht, so nimmt die Dosierhülse 1 mittels der Kopplung 1a, 2a die Anzeigetrommel 2 mit, welche sich beim Aufdosieren in proximale Richtung durch das Gewinde 2b, 3a geführt in das Gehäuse 3 einschraubt.

In dem Gehäuse 3 ist ein Sichtfenster 3b vorgesehen, an welchem eine auf der Außenseite der Anzeigetrommel 2 angebrachte Beschriftung, an welcher die eingestellte Dosis abgelesen werden kann, dargestellt wird und abgelesen werden kann.

Die Anzeigetrommel 2 weist innen liegend die Funktion einer an sich bekannten Drehhülse auf, wobei ein radial nach innen vorgespannter Schnapparm 2c vorgesehen ist, welcher in die Verzahnung 4a einer innerhalb der Anzeigetrommel 2 geführten Zahn-Gewindestange 4 eingreift, wobei der Schnapparm 2c im Zusammenwirken mit der Verzahnung 4a oder einem Zahngewinde der Zahngewindestange 4 so ausgebildet ist, dass beim Aufdosieren, bei welchem die Zahngewindestange 4 von einer Führungshülse 5 mittels eines oder mehrerer radial nach innen vorgespannter Schnapparme 5c, welche in das verzahnte Gewinde 4b der Zahngewindestange 4 eingreifen, gehalten wird, der Schnapparm 2c außer Eingriff gebracht werden kann, so dass sich die Anzeigetrommel 2 relativ zur Zahngewindestange 4 drehen kann, wobei sich der Schnapparm 2c um mehrere Klicks oder Zahnungen im Umfang um die Zahngewindestange 4 dreht. Eine Drehbewegung in Gegenrichtung wird jedoch durch den Schnapparm 2c verhindert. Die Schnapparme 5c der Führungshülse 5 können prinzipiell wie die Schnapparme 2c der Anzeigetrommel 2, ausgebildet sein und zum Beispiel an Federarmen angebrachte sich in radiale Richtung erstreckende Rastelemente aufweisen, wirken jedoch bezüglich der Zahngewindestange 4 in entgegengesetzte Richtung.

Eine Federspannhülse 6 ist axial nicht verschiebbar aber drehbar in der Injektionsvorrichtung bzw. in dem Gehäuse 3 gelagert. Auf der Außenseite der Federspannhülse 6 sind radial nach außen vorstehende Stege 6a vorgesehen, welche in radial verlaufende Führungsnuten 2d auf der Innenseite der Anzeigetrommel 2 eingreifen, so dass eine Drehbewegung der Anzeigetrommel 2 auf die Federspannhülse 6 übertragen werden kann. Die Anzeigetrommel 2 ist relativ zur Federspannhülse 6 verdrehgesichert, jedoch relativ dazu in axiale Richtung verschiebbar.

Auf der Innenseite im proximalen Bereich der Federspannhülse 6 ist eine Spiralfeder 7 vorgesehen, welche wie eine an sich bekannte Torsionsfeder, Schneckenfeder, gewickelte Blattfeder oder Uhrenfeder ausgebildet sein kann. Die Spiralfeder 7 ist an der äußeren Seite mit der Federspannhülse 6 verbunden und an der Innenseite mit der Führungshülse 5 verbunden.

Beim Aufdosieren, d.h. beim Drehen der Dosierhülse 1, ist die Führungshülse 5 durch einen oder mehrere radial nach außen vorgespannte Schnapparme 5a, welche in eine Innenverzahnung 3d des Gehäuses 3 eingreifen, relativ zur Injektionsvorrichtung bzw. zum Gehäuse 3 verdrehgesichert, so dass bei einer Drehbewegung der Dosierhülse 1 die Spiralfeder 7 aufgezogen wird. Ebenso wie die Schnapparmverbindungen 2c, 4a und 5c, 4b bewirkt die Schnapparmkopplung 5a, 3d nur eine Verdrehsicherung in eine Richtung und gibt die Gegendrehbewegung frei.

Anders als bei den bekannten Pens ist die Führungshülse 5 somit nicht immer verdrehsicher mit dem Pen bzw. dem Gehäuse 3 gekoppelt, sondern ist in eine Richtung drehbar, welche der Richtung entgegengesetzt ist, die zum Aufdosieren bzw. zum Einstellen der Dosis mittels der Dosierhülse 1 verwendet wird. Die Führungshülse 5 weist in einer bevorzugten Ausführungsform zwei einander gegenüberliegende radial nach außen vorgespannte Schnapparme 5a auf, welche in die Innenverzahnung oder Längsverzahnung 3c des Gehäuses 3 eingreifen. Durch die Schnapparme 5a kann die Führungshülse 5 somit nur in eine durch das Schnapparmprofil vorgegebene Drehrichtung gedreht werden, wohingegen eine Drehung in die Gegenrichtung blockiert wird. An der Innenseite hat die Führungshülse 5 ein Innengewinde 5b, in welchem das Außengewinde 4b der Zahngewindestange 4 geführt wird. Die an der Führungshülse 5 vorgesehenen Schnapparme 5c bilden ein auskoppelbares Verdrehsicherungselement und greifen in die Zahnung des Zahngewindes 4b der Zahngewindestange 4 ein, so dass der Schnapparm 5c mit der Zahngewindestange 4 gekoppelt ist und Zahngewindestange 4 und Führungshülse 5 eine Einheit bilden.

Ist durch Drehung der Dosierhülse 1 eine zu große Dosis eingestellt worden, so kann die Dosierhülse 1 zur Verkleinerung der Dosis zurückgedreht oder korrigiert werden. Beim Zurückdrehen bildet die Dosierhülse 1, die Anzeigetrommel 2, die Federspannhülse 6, die Zahngewindestange 4 und die Führungshülse 5 eine verdrehgesicherte Einheit. Insbesondere ist die Dosierhülse 1 mittels der Verdrehsicherung 1a, 2a und des Schnapparmes 2c der Anzeigetrommel 2 verdrehgesichert mit der Zahngewindestange 4 gekoppelt, welche beim Zurückdrehen der Dosierhülse 1 ebenfalls zurückgedreht wird. Bei dieser Zurückdrehbewegung verschiebt sich jedoch die Zahngewindestange 4 nicht in axiale Richtung. Mittels der Schnapparme 5c ist die Führungshülse 5 verdrehgesichert mit der Zahngewindestange 4 gekoppelt und wird somit bei der Rückdrehbewegung von der Zahngewindestange 4 mitgenommen. Dabei dreht sich die Führungshülse 5 relativ zu dem Gehäuse 3 der Injektionsvorrichtung.

Dabei bleibt die Spiralfeder 7 auf Grund der sich nicht relativ drehenden Führungshülse 5 und Federspannhülse 6 gespannt. Würde die Injektionsvorrichtung durch Hin- und Herdrehen der Dosierhülse 1 zum Einstellen und Korrigieren einer Dosis mehrfach betätigt, so würde sich die Spiralfeder 7 immer weiter spannen.

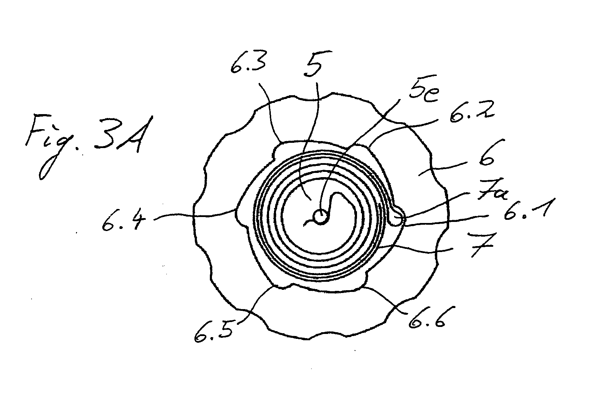

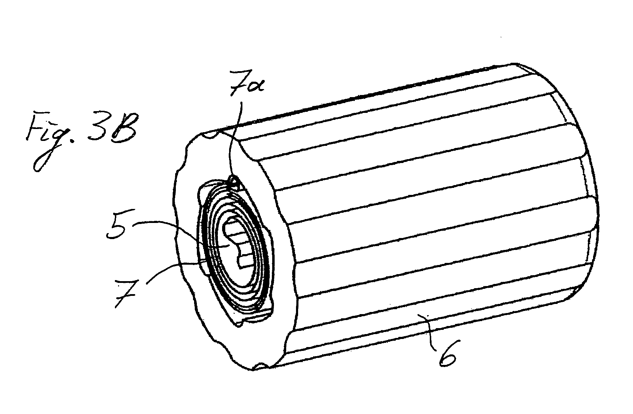

Um zu verhindern, dass die Spiralfeder 7 überspannt wird, wird der Umstand ausgenutzt, dass eine Spiralfeder 7, welche in der Mitte bzw. auf der Innenseite an einer Federhalterung 5e der Führungshülse 5 festgehalten wird, beim Spannen einen zunehmend kleineren Außendurchmesser aufweist. Im äußeren Endbereich weist die Spiralfeder 7 ein Kupplungselement 7a auf, wie in Fig. 3 gezeigt, welches in einer von mehreren möglichen Haltepositionen 6.1, 6.2, 6.3, 6.4, 6.5 oder 6.6 an der Innenseite der Federspannhülse 6 gehalten wird. Die Haltepositionen 6.1 bis 6.6 können zum Beispiel durch axial verlaufende Nuten gebildet werden, in welche eine Ausbiegung oder Ausbuchtung oder ein verdicktes Ende 7a am äußeren Ende der Spiralfeder 7 eingreift. Ist die Spiralfeder 7 so weit aufgezogen, dass sich der Außendurchmesser so weit verkleinert hat, dass die äußere Ausbuchtung 7a der Spiralfeder 7 sich aus der in axiale Richtung verlaufenden Nut 6.1 der Federspannhülse 6 zurückzieht, so entspannt sich die Spiralfeder 7 selbsttätig, wodurch das Halteelement 7a um eine Halteposition zu der Position 6.2 innerhalb der Federspannhülse 6 weiterrutscht. Somit kann ein Überspannen der Spiralfeder 7 durch den oben geschilderten Selbstentspannungsmechanismus verhindert werden.

Zum Auslösen wird ein Auslöseknopf 8 gegen die Kraft einer axial wirkenden Feder 9 in distale Richtung der Injektionsvorrichtung eingedrückt. Auf der Innenseite weist der Auslöseknopf 8 in axiale Richtung nach innen vorstehende Arme oder einen Zylinder 8a auf, welcher an seiner vorderseitigen Stirnfläche konisch zulaufend ausgebildet ist. Der konische Bereich des Zylinders 8a greift an einer schrägen Anlagefläche 5d der Schnapparme 5c der Führungshülse 5 ein, wie in Figur 1B gezeigt, und hebt diese radial nach außen an, so dass die Kopplung zwischen den Schnapparmen 5c und der Zahngewindestange 4 gelöst wird.

Die Führungshülse 5 ist beim Ausschütten drehfest mit dem Gehäuse 3 der Injektionsvorrichtung durch die Schnapparme 5a verbunden. Die zwischen der Führungshülse 5 und der Federspannhülse 6 gespannte Spiralfeder 7 erzeugt somit ein Drehmoment auf der Federspannhülse 6, welche das Drehmoment auf die verdrehsicher gekoppelte Anzeigetrommel 2 und die mit der Anzeigetrommel 2 verdrehgesicherte Dosierhülse 1 überträgt, wobei die Drehbewegung der Anzeigetrommel 2 mittels des Schnapparmes 2c auf die Zahngewindestange 4 übertragen wird, welche sich geführt durch das Innengewinde 5b der Führungshülse 5 relativ zur Führungshülse 5 in distale Richtung einschraubt und somit eine Vorschubbewegung erzeugt. Beim Entspannen der Spiralfeder 7 wird auch gleichzeitig die Anzeigetrommel 2 zurückgedreht.

Anschließend kann mittels der Dosierhülse 1 wieder mit einem neuen Einstellvorgang begonnen werden.

Ergänzend wird angemerkt, dass die beschriebene Dosier- und Injektionsvorrichtung zum dosierten Ausschütten einer Dosis aus einer normalen Ampulle oder einer Zwei-Kammer-Ampulle 10, wie in Figur 2 gezeigt, verwendet werden kann. Dabei wird die Zwei-Kammer-Ampulle 10 in die Injektionsvorrichtung vor dem Abgeben einer Dosis auf bekannte Art eingeschraubt, wodurch der proximale Stopfen 10a in Richtung auf den distalen Stopfen 10b verschoben wird, um das in dem Volumen zwischen den Stopfen 10a und 10b vorhandene Fluid mit dem am distalen Ende des Stopfens 10b gelagerten Fluid vor dem Beginn einer Injektion abzumischen.

## Patentansprüche

1. Dosiervorrichtung für eine Injektionsvorrichtung mit einem Dosierelement (1), welches zur Einstellung einer Dosis in eine erste Richtung beweget oder gedreht werden kann und mit einem Führungselement (5) zum Führen einer Gewindestange (4), wobei das Dosierelement (1) zum Einstellen einer Dosis relativ zum Führungselement (5) beweget oder gedreht wird, **dadurch gekennzeichnet, dass** das Führungselement (5) bei einer Bewegung oder Drehung des Dosierelementes (1) in Gegenrichtung zur ersten Richtung zur Korrektur oder Verringerung der eingestellten Dosis mitgedreht oder mitbewegt wird.

2. Dosiervorrichtung nach Anspruch 1 mit einer Zahn-Gewindestange (4) mit einem verzahnten Gewinde (4b) oder verzahnten Gewindeabschnitt und/oder einem verzahnten Bereich (4a).

3. Dosiervorrichtung nach einem der vorhergehenden Ansprüche mit einem Koppelelement oder Anzeigeelement (2), welches verdrehgesichert (1a, 2a) mit dem Dosierelement (1) gekoppelt ist und welches einen einseitig wirkenden Verdrehsicherungsmechanismus (2c) aufweist, um eine Verdrehsicherung des Koppelelementes (2) mit der Gewindestange (4) in eine Drehrichtung zu realisieren, wobei eine Drehung in Gegenrichtung möglich ist.

4. Dosiervorrichtung nach einem der vorhergehenden Ansprüche, wobei das Koppelelement (2) ein Gewinde (2b) aufweist, welches in der Dosiervorrichtung oder einer Injektionsvorrichtung (3, 3a) geführt werden kann.

5. Dosiervorrichtung nach einem der vorhergehenden Ansprüche mit einem Federspannelement (6), welches mit dem Koppelelement (2) und / oder dem Dosierelement (1) verdrehsicher gekoppelt ist.

6. Dosiervorrichtung nach einem der vorhergehenden Ansprüche mit einem Federelement (7), welches zwischen dem Führungselement (5) und dem Federspannelement (6) gehalten wird.

7. Dosiervorrichtung nach dem vorhergehendem Anspruch, wobei das Federelement (7) mit dem Führungselement (5) oder mit dem Federspannelement (6) lösbar oder unlösbar verbunden ist.

8. Dosiervorrichtung nach einem der vorhergehenden Ansprüche, wobei das Führungselement (5) mindestens ein einseitig gerichtetes Drehsicherungselement (5c) aufweist, um eine Relativbewegung oder Relativdrehung der Gewindestange (4) in eine Richtung zu ermöglichen und in Gegenrichtung zu sperren.

9. Dosiervorrichtung nach einem der vorhergehenden Ansprüche, wobei das Führungselement (5) mindestens ein Verdrehsicherungselement (5a) aufweist, um eine Drehung des Führungselementes (5) relativ zur Dosiervorrichtung oder relativ zu einer Injektionsvorrichtung in eine Richtung zu ermöglichen und in Gegenrichtung zu sperren.

10. Dosiervorrichtung nach einem der vorhergehenden Ansprüche mit einem Auslöseelement (8), mit welchem die Verdrehsicherung (5c, 4b) zwischen dem Führungselement (5) und der Gewindestange (4) gelöst werden kann.

11. Injektionsvorrichtung mit einer Dosiervorrichtung nach einem der vorgehenden Ansprüche.

12. Verfahren zum Korrigieren einer mit einer Dosiervorrichtung (1, 5) eingestellten Dosis, wobei die Dosis durch Bewegung oder Drehung eines Dosierelements (1) relativ zu einem Führungselement (5) eingestellt wird und wobei das Führungselement (5) beim Korrigieren der Dosis durch Bewegen oder Drehen des Dosierelementes (1) in Gegenrichtung mit dem Dosierelement (1) bewegt oder mitgedreht wird.

## Claims

1. A dosing device for an injection device comprising a dosing element (1) which for setting a dose can be moved or rotated in a first direction and a guide element (5) for guiding a threaded rod (4), wherein for setting a dose the dosing element (1) is moved or rotated relative to the guide element (5), **characterised in that** the guide element (5) is also rotated or moved upon a movement or rotation of the dosing element (1) in the opposite direction to the first direction for correcting or reducing the set dose.

2. A dosing device according to claim 1 having a toothed threaded rod (4) with a toothed thread (4b) or toothed thread portion and/or a toothed region (4a).

3. A dosing device according to one of the preceding claims comprising a coupling element or display element (2) which is coupled to the dosing element (1) in non-rotational relationship (1a, 2a) and which has a rotation securing mechanism (2c) acting at one side to provide for rotational locking of the coupling element (2) to the threaded rod (4) in a direction of rotation, wherein rotation in the opposite direction is possible.

4. A dosing device according to one of the preceding claims wherein the coupling element (2) has a thread (2b) which can be guided in the dosing device or an injection device (3, 3a).

5. A dosing device according to one of the preceding claims comprising a spring stressing element (6) which is non-rotatably coupled to the coupling element (2) and/or the dosing element (1).

6. A dosing device according to one of the preceding claims comprising a spring element (7) held between the guide element (5) and the spring stressing element (6).

7. A dosing device according to the preceding claim wherein the spring element (7) is releasably or non-releasably connected to the guide element (5) or to the spring stressing element (6).

8. A dosing device according to one of the preceding claims wherein the guide element (5) has at least one rotation locking element (5c) directed at one side to permit a relative movement or relative rotation of the threaded rod (4) in one direction and to block it in the opposite direction.

9. A dosing device according to one of the preceding claims wherein the guide element (5) has at least one rotation locking element (5a) to permit a rotation of the guide element (5) relative to the dosing device or relative to an injection device in one direction and to block it in the opposite direction.

10. A dosing device according to one of the preceding claims comprising a triggering element (8) with which the rotational lock (5c, 4b) between the guide element (5) and the threaded rod (4) can be released.

11. An injection device having a dosing device according to one of the preceding claims.

12. A method of correcting a dose set with a dosing device (5), wherein the dose is set by movement or rotation of a dosing element (1) relative to a guide element (5) and wherein the guide element (5) is moved or rotated with the dosing element (1) upon correction of the dose by moving or rotating the dosing element (1) in the opposite direction.

## Revendications

1. Dispositif de dosage pour un système d'injection comprenant un élément de dosage (1), qui peut être déplacé ou tourné dans une première direction pour le réglage d'une dose et comprenant un élément de guidage (5) pour le guidage d'une tige filetée (4), dans lequel l'élément de dosage (1) est déplacé ou tourné par rapport à l'élément de guidage (5) pour le réglage d'une dose, **caractérisé en ce que** l'élément de guidage (5) est tourné ou déplacé conjointement lors d'un déplacement ou d'une rotation de l'élément de dosage (1) dans la direction opposée à la première direction pour corriger ou réduire la dose réglée.

2. Dispositif de dosage selon la revendication 1 comprenant une tige filetée dentée (4) avec un filetage denté (4b) ou une section de filetage denté et/ou une zone dentée (4a).

3. Dispositif de dosage selon l'une des revendications précédentes comprenant un élément de couplage ou un élément d'affichage (2), qui est couplé à l'élément de dosage (1) de manière à résister à la torsion (la, 2a) et qui présente un mécanisme de résistance vis-à-vis de la torsion (2c) ayant une action unilatérale pour réaliser une résistance à la rotation de l'élément de couplage (2) avec la tige filetée (4) dans une direction de rotation, dans lequel une rotation dans la direction inverse est possible.

4. Dispositif de dosage selon l'une des revendications précédentes, dans lequel l'élément de couplage (2) présente un filetage (2b) qui peut être guidé dans le dispositif de dosage ou dans un système d'injection (3, 3a).

5. Dispositif de dosage selon l'une des revendications précédentes comprenant un élément de contrainte à ressort (6) qui est couplé de manière résistante à la torsion avec l'élément de couplage (2) et/ou l'élément de dosage (1).

6. Dispositif de dosage selon l'une des revendications précédentes comprenant un élément à ressort (7) qui est maintenu entre l'élément de guidage (5) et l'élément de contrainte à ressort (6).

7. Dispositif de dosage selon l'une des revendications précédentes, dans lequel l'élément à ressort (7) est lié à l'élément de guidage (5) ou à l'élément de contrainte à ressort (6) de manière amovible ou inamovible.

8. Dispositif de dosage selon l'une des revendications précédentes, dans lequel l'élément de guidage (5) présente au moins un élément de résistance à la rotation (5c) dirigé unilatéralement pour permettre un déplacement relatif ou une rotation relative de la tige filetée (4) dans une direction et le/la bloquer dans la direction inverse.

9. Dispositif de dosage selon l'une des revendications précédentes, dans lequel l'élément de guidage (5) présente au moins un élément de résistance à la torsion (5a) pour permettre une rotation de l'élément de guidage (5) par rapport au dispositif de dosage ou par rapport à un système d'injection dans une direction et la bloquer dans la direction inverse.

10. Dispositif de dosage selon l'une des revendications précédentes, comprenant un élément de dégagement (8), grâce auquel la résistance à la torsion (5c, 4b) entre l'élément de guidage (5) et la tige filetée (4) peut être supprimée.

11. Système d'injection comprenant un dispositif de dosage selon l'une des revendications précédentes.

12. Procédé pour la correction d'une dose réglée avec un dispositif de dosage (1, 5), dans lequel la dose est réglée par déplacement ou rotation d'un élément de dosage (1) par rapport à un élément de guidage (5) et dans lequel l'élément de guidage (5) est déplacé avec l'élément de dosage (1) ou tourné conjointement dans la direction inverse lors de la correction de la dose par déplacement ou rotation de l'élément de dosage (1).
